# EUROPEAN PATENT APPLICATION

(11) **EP 4 571 306 A1**
(43) Date of publication of application: **18.06.2025**
(21) Application number: 24217632.9
(22) Date of filing: 05.12.2024
(51) Int. Cl.: G01N 33/49

(54) **STABILIZING OXYGEN IN A COMPOSITION**

(30) Priority: 11.12.2023 US 202318535453; 12.01.2024 LU 506091
(71) Applicant: Instrumentation Laboratory Company, Bedford, MA 01730 (US)
(72) Inventor: ERDOSY, Miklos, Bedford, 01730 (US); BOUZIANE, Nesrine, Bedford, 01730 (US); DEMIRDJIAN, Jacqueline, Bedford, 01730 (US); PAMIDI, Prasad, Bedford, 01730 (US)
(74) Representative: Müller-Boré & Partner Patentanwälte PartG mbB

(57) **Abstract**

A composition includes one or more analytes for use with an electrochemical sensor configured to detect a component of a biological sample, and a quinolones at a concentration of 60 milligrams/liter (mg/L) to 120 mg/L in the composition, to stabilize oxygen in a presence of trace metal.

## Description

### TECHNICAL FIELD

This specification relates generally to diagnostic testing and devices, test kits, containers, and cartridges used during diagnostic testing.

### BACKGROUND

Molecular or dioxygen is a reactive component in multi-analyte solutions routinely used in test systems, such as blood gas analyzers. An oxidation reaction is a process in which a chemical substance formally loses electrons and can be directly or indirectly mediated by molecular oxygen, with or without the incorporation of oxygen in some form into the chemical substance. For example, oxidation of organic compounds or biomolecules (autooxidation) can be catalyzed by some metal ions, and some metal ions can be directly oxidized by molecular oxygen. When a substance, such as metal, is oxidized, a result is a lesser amount of molecular oxygen in a composition than before the oxidation occurred.

### SUMMARY

This specification relates generally to stabilizing molecular oxygen in a composition by incorporating a chelating agent such as quinolones, into the composition.

An example composition includes one or more analytes for use with an electrochemical sensor configured to detect a component of a biological sample, and a quinolones at a concentration of 60 milligrams/liter (mg/L) to 120 mg/L in the composition, to stabilize oxygen in a presence of trace metal. The example composition may include one or more of the following features.

The quinolones may be or include a fluoroquinolone. The quinolones may be or include levofloxacin. The quinolones may be or include at least one of: ciprofloxacin, gemifloxacin, moxifloxacin, norfloxacin, ofloxacin, pefloxacin, enoxacin, amifloxacin, tosufloxacin, difloxacin, temafloxacin, miloxacin, cinoxacin, lomefloxacin, fleroxacin, fluoroquinolones, nalidixic acid, pipemidic acid, piromidic acid, rosoxacin, oxolinic acid, flumequine, enrofloxacin, sparfloxacin, gatifloxacin, balofloxacin, clinafloxacin, or sitafloxacin.

The composition may include an aqueous liquid. The composition may include a trace metal. In particular, the composition may include one or more trace metals. The trace metal(s), in particular the trace metal(s) in the composition, may include or consist of one or more metals. The one or more metals may be at a concentration in the composition of 10 parts-per-billion (ppb) or more. The aqueous liquid may contain dissolved oxygen. The one or more analytes may include: pH (hydronium), carbon dioxide (CO₂), oxygen (O₂), sodium (Na), potassium (K), chlorine (CI), calcium (Ca), magnesium (Mg), glucose, lactate, creatinine, creatinine, urea, organic dyes, amaranth, or sulforhodamine B. The dissolved oxygen may be at a known concentration in the composition.

A container may hold the composition that includes one or more analytes for use with an electrochemical sensor configured to detect a component of a biological sample, and a quinolones at a concentration of 60 milligrams/liter (mg/L) to 120 mg/L in the composition, to stabilize oxygen in the presence of trace metal. The container may be or include a bag, pouch, or glass. The container may be or include a cartridge.

An example system includes a cartridge including multiple containers. Each of the multiple containers holds a composition. Each composition includes: a liquid including one or more analytes, and a quinolones at a concentration of 60 milligrams/liter (mg/L) to 120 mg/L in the composition. Liquids in different ones of the containers have different concentrations of the one or more analytes. The example system also includes an electrochemical sensor configured to detect at least one of the analytes. The electrochemical sensor may be internal to, or external to, the cartridge. The example system may include one or more of the following features, either alone or in combination.

The quinolones may be or include levofloxacin. The quinolones may be or include at least one of: ciprofloxacin, gemifloxacin, moxifloxacin, norfloxacin, ofloxacin, pefloxacin, enoxacin, amifloxacin, tosufloxacin, difloxacin, temafloxacin, miloxacin, cinoxacin, lomefloxacin, fleroxacin, fluoroquinolones, nalidixic acid, pipemidic acid, piromidic acid, rosoxacin, oxolinic acid, flumequine, enrofloxacin, sparfloxacin, gatifloxacin, balofloxacin, clinafloxacin, or sitafloxacin.

The composition may include one or more metals. The one or more metals may be at a concentration in a respective composition of 10 parts-per-billion (ppb) or more. The composition may include an aqueous liquid. The aqueous liquid may contain dissolved oxygen. The dissolved oxygen may be one of the analytes. The one or more analytes may include: pH (Hydronium), carbon dioxide (CO₂), oxygen (O₂), sodium (Na), potassium (K), chlorine (CI), calcium (Ca), magnesium (Mg), glucose, lactate, organic dyes, sulforhodamine B, or amaranth.

An example kit includes a quinolones and an aqueous composition including one or more analytes for use with an electrochemical sensor configured to detect a component of a biological sample.

An example method performs oxygen measurement in a composition that includes one or more analytes for use with an electrochemical sensor configured to detect a component of a biological sample, and a quinolones at a concentration of 60 milligrams/liter (mg/L) to 120 mg/L in the composition, to stabilize oxygen in the presence of trace metal. The example method includes contacting a surface of an inert metal electrode to the composition, reducing oxygen at the surface, and producing an electrical signal that is detected by a clinical analyzer. The quinolones stabilizes oxygen in the composition prior to the oxygen measurement. The example method may include one or more of the following features, either alone or in combination.

The quinolones may optionally be or include levofloxacin. The quinolones may optionally be or include at least one of: ciprofloxacin, gemifloxacin, moxifloxacin, norfloxacin, ofloxacin, pefloxacin, enoxacin, amifloxacin, tosufloxacin, difloxacin, temafloxacin, miloxacin, cinoxacin, lomefloxacin, fleroxacin, fluoroquinolones, nalidixic acid, pipemidic acid, piromidic acid, rosoxacin, oxolinic acid, flumequine, enrofloxacin, sparfloxacin, gatifloxacin, balofloxacin, clinafloxacin, or sitafloxacin. The one or more analytes optionally may include: pH (hydronium), carbon dioxide (CO₂), oxygen (O₂), sodium (Na), potassium (K), chlorine (Cl), calcium (Ca), magnesium (Mg), glucose, lactate, creatine, creatinine, urea, organic dyes, amaranth, or sulforhodamine B. Optionally, the composition may be contained in a sealed container prior to the oxygen measurement. Optionally, the composition may include one or more metals optionally at a concentration of 10 parts-per-billion (ppb) or more.

Features, embodiments and effects as particularly described herein with respect to the composition, with respect to the container, with respect to the system, with respect to the kit and/or with respect to the method accordingly apply to respective other ones of the container, the system, the kit and/or the method.

The compositions, devices, systems, and processes described in this specification may be configured, for example, through design, construction, formulation, arrangement, placement, programming, operation, activation, deactivation, and/or control.

Two or more of the features described in this specification, including in this summary section, may be combined to form implementations not specifically described in this specification.

The details of one or more implementations are set forth in the accompanying drawings and the description below. Other features and advantages will be apparent from the description and drawings, and from the claims.

### DESCRIPTION OF THE DRAWINGS

Fig. 1 is a block diagram of an example container containing an example composition containing metal contaminants and a chelating agent.
Fig. 2 is block diagram illustrating an example chelating process conceptually.
Fig. 3 is a block diagram of components for an example test system that includes an example composition containing a chelating agent.

Like reference numerals indicate like elements.

### DETAILED DESCRIPTION

An example test system uses a liquid composition containing an aqueous liquid such as deionized (DI) water or the buffers described herein, and a known, predefined concentration of dissolved oxygen. The composition may be stored in a tightly sealed, substantially gas-tight container, such as a bag, pouch, or glass bottle. The dissolved oxygen may be among analytes to be detected by an electrochemical sensor (referred to herein simply as a "sensor") in the test system. "Oxygen" in this context and as used herein refers to molecular, dioxygen, or free oxygen, which includes oxygen atoms not attached to another element.

The composition may be part of a calibration solution or control solution. For example, the test system may use the composition to calibrate the sensor. In an example, the test system may detect a concentration of oxygen in the composition and compare the concentration of oxygen detected to the concentration of oxygen known to be in the composition. If the two concentrations differ, the test system may apply a calibration factor to measurements from the sensor.

The composition also contains traces of metal including metal ions. These traces of metal ions are contaminants that may have been introduced inadvertently by production equipment during manufacture of the container or composition or as contaminants in raw materials that are used to formulate the composition. To prevent, limit, or minimize oxidation of the metal ions, a chelating agent such as a quinolones is added to the composition in the container.

The chelating agent binds to the contaminant metal ions by forming stable complexes with these metal ions through two or more covalent or coordinate linkages. The effect is essentially to render the metal ions inert to oxygen, thereby preventing or limiting oxidation reactions in the composition and the concomitant consumption of oxygen. In general, in some implementations, other than the metal contaminants, the chelating agent does not react with any of the components of the composition. For example, the chelating agent may not chemically react with any appreciable or detectable amount of analytes in the composition, a test sample, or active components of the sensor, such as reagents included on the sensor.

Quinolones include quinolone-3-carboxylic acid and its analogues. Quinolones include a class of antibacterial drugs that are analogues of hydroxylated quinolines and that inhibit replication of bacteria by inhibiting DNA (deoxyribonucleic acid) replication. Quinolones can be particularly useful as a chelating agent in a testing context such as those as described herein, since quinolones bind strongly to metal contaminants, such as chromium, iron, or aluminum, but bind weakly, reversibly, or to no appreciable/detectable amount to analytes to be detected by sensors in the test system. Examples of such analytes to be detected include pH (hydronium), sodium, potassium, chloride, hydrogen ions, carbon dioxide, glucose, lactate, creatine, creatinine, organic dye, and/or alkaline earth metals such as calcium or magnesium. For example, a quinolones' binding strength to divalent ions follows the order: Fe3+ > AI3+ > Cu2+ > Fe2+ > Zn2+ > Mg2+ > Ca2+, where binding to magnesium and calcium ions is weak as compared to iron and aluminum ions. Strongly chelating the analytes to be detected would change their concentrations and, thereby, adversely affect measurements of these analytes by the sensor.

In contrast to quinolones, known chelating agents such as EDTA (Ethylenediaminetetraacetic acid), Tetraethylenetetraamine (TETA) and Nitrilotriacetic acid (NTA), strongly chelate calcium and, therefore, cannot be used to test certain types of analytes such as calcium.

Furthermore, by incorporating a chelating agent into the composition, the concentration of oxygen in the composition remains stable over a prolonged duration. Otherwise, direct, or mediated reactions with one or more trace metals consume the oxygen in the composition and alter the oxygen concentration, thereby causing the oxygen in the composition to deviate from the known, predefined concentration. The shelf-life of the container, which is the duration of time the container may be used for testing, may thus be increased relative to containers that do not contain a chelating agent in the composition. For example, the shelf-life of the container containing a chelating agent can exceed 9 months, 10 months, 11 months, 12 months, or more. Test results using the compositions in these containers can therefore remain accurate over the prolonged duration, and lead to a longer shelf life than without these compositions.

Still further, since the sensor is in contact with the composition, the sensor potentially could be affected by the chelating agent. However, the chelating agent(s) in the composition do not interfere with diffusion of analytes through outer or inner sensor membranes of the sensor. Example outer or inner sensor membranes that a chelating agent does not interfere with may include, but are not limited to, plasticized PVC membrane with valinomycin ionophore, or ETH 1001, which is a calcium ionophore used to measure potassium or calcium. Still further, chelating agent(s) in the composition do not react with enzymes in the sensor, such as glucose oxidase, lactate oxidase, creatininese, creatine amidinohydrolase, or sarcosine oxidase. Therefore, use of chelating agent in the composition does not affect operation of the sensor.

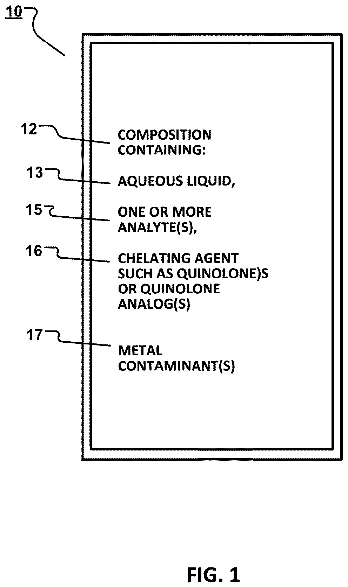

Fig. 1 shows an example of a container 10 that may be used to store an example composition 12 of the type described above. In this example, composition 12 includes an aqueous liquid 13, one or more analytes 15, one or more chelating agents 16, and traces of one or more types metal ion contaminants 17.

Examples of the aqueous liquid 13 may include, but are not limited to, DI water and a buffer or non-buffered aqueous liquid containing at least one of sodium chloride, potassium chloride, calcium chloride, glucose, or sodium lactate.

The one or more analytes 15 include oxygen (O₂), and may also include, but are not limited to, one or more of the following: pH (hydronium), carbon dioxide (CO₂), sodium (Na), potassium (K), chlorine (CI), calcium (Ca), magnesium (Mg), glucose (Glu), lactate (Lac), creatine, creatinine, urea, organic dyes, amaranth, and sulforhodamine B. It is understood that the analytes can be in an ionic or neutral form, depending on their stability and equilibrium state in the solution. The one or more analytes, including the O₂, may be dissolved in the aqueous liquid.

The metal contaminants 17 are not an intended component of the composition, but rather are a contaminant. The metal contaminants may be traces of metal at low concentrations, which may have concentrations in the double-digital parts-per-million (ppm) or parts-per-billion (ppb) range. Examples of metal that may make up the contaminants include, but are not limited to, chromium, iron, aluminum, cobalt, manganese, and copper. Metal atoms tend to hold their outer electrons relatively more loosely than their inner electrons. Therefore, metal atoms tend to lose electrons. This loss of electrons results in metal ions in the composition in various oxidation states which, absent a chelating agent as described herein, combine with the oxygen during an oxidation reaction, thereby depleting oxygen in the composition. Metals in lower oxidation states, such as the elemental forms in flakes of steel or the like, can also corrode and react with oxygen, depleting oxygen in the solution.

Examples of chelating agents 16 that may be included in composition include, but are not limited to, compounds such as quinolones. Examples of quinolones that may be used in composition 12 as chelating agents include, but are not limited to, one or more of levofloxacin, ciprofloxacin, gemifloxacin, moxifloxacin, norfloxacin, ofloxacin, pefloxacin, enoxacin, amifloxacin, tosufloxacin, difloxacin, temafloxacin, miloxacin, cinoxacin, lomefloxacin, fleroxacin, fluoroquinolones, nalidixic acid, pipemidic acid, piromidic acid, rosoxacin, oxolinic acid, flumequine, enrofloxacin, sparfloxacin, gatifloxacin, balofloxacin, clinafloxacin, or sitafloxacin. In some implementations the chelating agents include one or more of ciprofloxacin, gemifloxacin, moxifloxacin, norfloxacin, ofloxacin, pefloxacin, enoxacin, amifloxacin, tosufloxacin, difloxacin, temafloxacin, miloxacin, cinoxacin, lomefloxacin, fleroxacin, fluoroquinolones, nalidixic acid, pipemidic acid, piromidic acid, rosoxacin, oxolinic acid, flumequine, enrofloxacin, sparfloxacin, gatifloxacin, balofloxacin, clinafloxacin, or sitafloxacin. In some implementations the chelating agent includes levofloxacin alone or in combination with other chelating agents. In some implementations, the composition may include a single quinolones. In some implementations, the composition may include a combination of two or more quinolones. In some implementations, a combination of different ones of the listed (or other) quinolones may be used to chelate different metal contaminants.

In some implementations, chelating agent(s) 16 is/are at a total concentration of greater than 60 milligrams/liter (mg/L) in the composition 12, at a total concentration of greater than 65 mg/L in the composition 12, at a total concentration of greater than 70 mg/L in the composition 12, at a total concentration of greater than 75 mg/L in the composition 12, at a total concentration of greater than 80 mg/L in the composition 12, at a total concentration of greater than 85 mg/L in the composition 12, at a total concentration of greater than 90 mg/L in the composition 12, at a total concentration of greater than 95 mg/L in the composition 12, at a total concentration of greater than 100 mg/L in the composition 12, at a total concentration of greater than 105 mg/L in the composition 12, at a total concentration of greater than 110 mg/L in the composition 12, or at a total concentration of greater than 115 mg/L in the composition 12.

In some implementations, chelating agent(s) 16 is/are at a total concentration of less than 200 mg/L in the composition 12, at a total concentration of 60 mg/L to 120 mg/L in the composition 12, at a total concentration of 65 mg/L to 120 mg/L in the composition 12, at a total concentration of 70 mg/L to 120 mg/L in the composition 12, at a total concentration of 75 mg/L to 120 mg/L in the composition 12, at a total concentration of 80 mg/L to 120 mg/L in the composition 12, at a total concentration of 85 mg/L to 120 mg/L in the composition 12, at a total concentration of 90 mg/L to 120 mg/L in the composition 12, at a total concentration of 95 mg/L to 120 mg/L in the composition 12, at a total concentration of 100 mg/L to 120 mg/L in the composition 12, at a total concentration of 105 mg/L to 120 mg/L in the composition 12, at a total concentration of 110 mg/L to 120 mg/L in the composition 12, at a total concentration of 115 mg/L to 120 mg/L in the composition 12, at a total concentration of 60 mg/L to 80 mg/L in the composition, at a total concentration of 80 mg/L to 100 mg/L in the composition, or at a total concentration of 100 mg/L to 120 mg/L in the composition. In some implementations, chelating agent(s) 16 is/are at a total concentration up to 200 mg/L in the composition 12. Other implementations may contain different total concentrations of quinolone(s) and/or quinolone analog(s) than those presented herein.

Varying levels of metal contamination can lead to variable rates of oxygen consumption in composition 12. The foregoing concentrations of chelating agents 16 in the composition 12 may be advantageous because it/they enable(s) chelation without affecting detection of the analyte. More specifically, significantly lower concentrations of quinolones 16 than those listed may not chelate, or may not chelate enough, of the metal contaminants in the composition to effectively prevent consumption of oxygen, particularly where there are greater concentrations of metal contaminants in the composition, such as concentrations in the single- or double-digit parts-per million (ppm) range. Significantly greater concentrations of chelating agents 16 than those listed may affect the behavior of a sensor used in the test system to detect the analyte. For example, significantly greater concentrations can change Ca and Mg concentrations in the composition and can inhibit enzyme function of enzyme-based sensors or alter the composition and function of ion-selective sensors.

The concentrations of chelating agents 16 listed may prevent, limit, or minimize oxidation in compositions containing metal contaminants at a concentration of 10 parts-per-billion (ppb) or more, at a concentration of 20 ppb or more, at a concentration of 30 ppb or more, at a concentration of 40 ppb or more, or at a concentration of 50 ppb or more, for example. In examples where the chelating agent is levofloxacin within one of the ranges of concentrations listed above in the composition, the levofloxacin may prevent or limit oxidation in compositions containing metal ions at a concentration of at least 10 ppb to 50 ppb, for example.

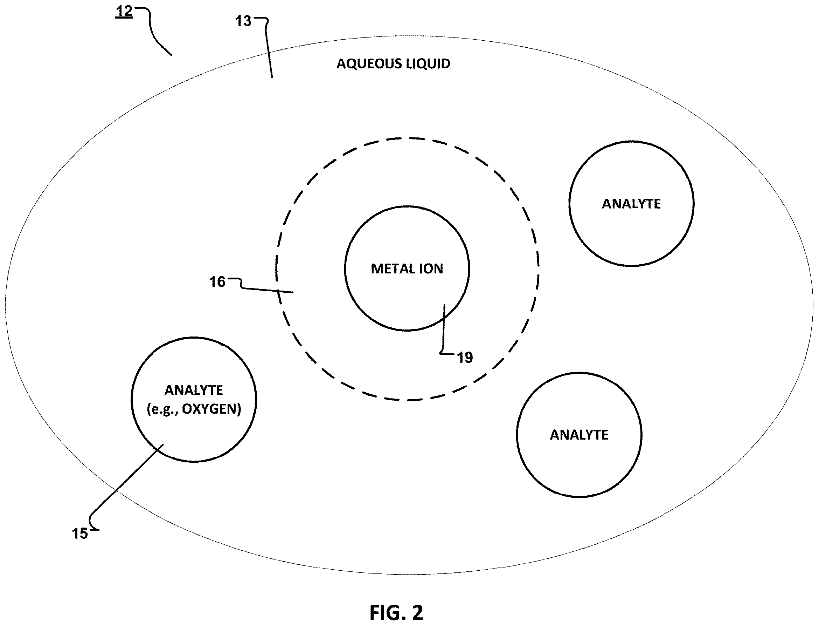

Fig. 2 shows composition 12 conceptually containing aqueous liquid 13, dissolved analyte 15, and metal ion 19 from metal contaminant 17. Chelating agent 16 binds to metal ion 19, preventing an occurrence of a reaction between metal ion 19 and oxygen, which is among the analytes 15 in this example.

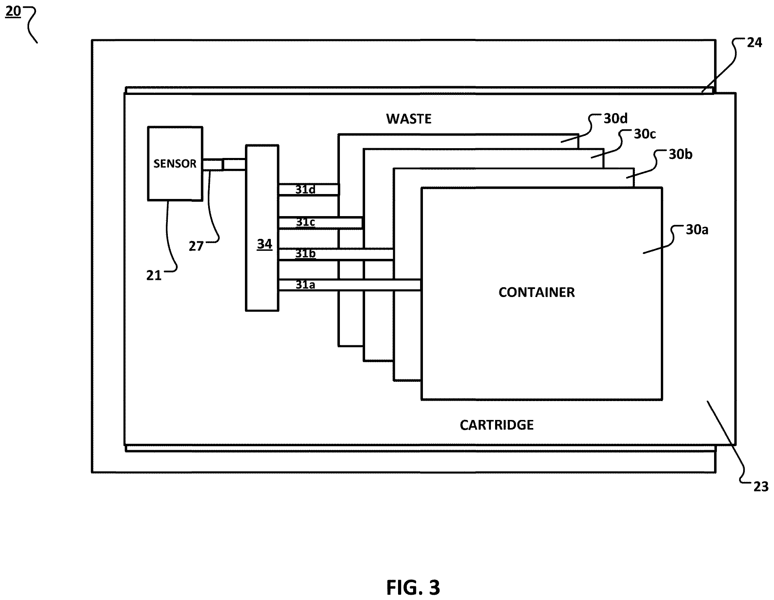

Fig. 3 shows an clinical analyzer 20, which is an example of a test system of the type described herein, and example cartridge 23 for use therewith. Clinical analyzer 20 may be configured to use a sensor to measure one or more analytes, such as those described herein, in composition 12 or in a test sample. Examples of the test sample include biological samples such as a bodily fluid, such as whole blood ("blood") or a component or derivative thereof. Examples of such components or derivatives include, but are not limited to, blood plasma and fluids containing red blood cells extracted from blood.

Clinical analyzer 20 includes a slot for receiving cartridge 23. In some implementations, clinical analyzer 20 may contain multiple slots (not shown), each for receiving a different instance of cartridge 23.

Cartridge 23 includes one or more containers 30a to 30d, each for holding an example of a composition 12, a sensor 21 for sensing an analyte in a test sample or in the composition, and ducts 31a to 31d for moving composition 12 from one of the containers 30a to 30d to sensor 21. Cartridge 23 may be a multi-use cartridge in some implementations. Cartridge 23 may be a single-use, disposable cartridge in some implementations.

Although four containers 30a to 30d are shown Fig. 3, cartridge 23 may contain fewer than four containers - for example, one, two, or three containers - or more than four containers - for example, five, six, seven, eight, and so forth containers. The containers may be sealed bags or pouches. The bags or pouches may be made of foil laminate, soft plastic, rubber, of any other type of soft liquid-tight and gas tight material. In some implementations, the containers may be made of hard plastic, glass, or any other type of hard liquid-tight and gas-tight material. Each container may be single-use, disposable container, or a multi-use container.

Each container may hold a composition 12 of the type described above with respect to Figs. 1 and 2. Different ones of the containers may hold compositions having different analytes. For example, in some implementations, each container 30a to 30d may contain the same known, predefined concentration of dissolved oxygen, but different other analytes, e.g., container 30a may contain calcium but not magnesium, and container 30b may contain magnesium but not calcium. Different ones of the containers may hold compositions having different concentrations of the same analytes. For example, each container 30a to 30d may hold a different known, predefined concentration of dissolved oxygen.

Clinical analyzer 20 may include one or more sensors 21 for performing tests on the compositions described herein and on test samples. In some implementations, as shown, the one or more sensors 21 may be part of one or more cartridges 23 received in clinical analyzer 20. In some implementations, the one or more sensors 21 are within clinical analyzer 20 not part of a cartridge 23 (not shown).

In some implementations, example sensor 21 contains one or more enzymes, analyte-specific ionophores, or inert metal electrodes such as platinum or gold, the composition of which can be specific to a particular analyte or analytes to be detected. An example of a sensor that may be used in clinical analyzer 20 is described with respect to Fig. 3 in U.S. Patent No. 6,872,297 (Mansouri), which issued on March 29, 2005, the contents of which are incorporated herein by reference.

Cartridge 23 includes example ducts 31a to 31d that are fluidically connectable to sensor 21 via example duct 27 to transport the compositions from respective containers 30a to 30d to cause the compositions to contact sensor 21.

During operation of clinical analyzer 20, a composition is moved (for example pumped) over sensor 21 from a container 30a to 30d via a corresponding duct. In an example where oxygen is to be detected, the sensor contains an oxygen probe, which can be an inert metal electrode, such as a platinum electrode. Oxygen measurement is performed on a surface of the metal electrode by reducing the oxygen to water or peroxide. That is, when the metal electrode comes into contact with the oxygen, there is reduction reaction between of the oxygen on the platinum surface, which produces water or hydrogen peroxide depending on the conditions. The electron transfer to the oxygen molecules causes an electrical signal on the platinum electrode, detected by an electrical detector of the clinical analyzer 20, which the clinical analyzer 20 uses to identify and to measure the concentration of oxygen in the composition. In some implementations, the electrical detector includes a voltage measurer. For example, the voltage measurer may be a hardware device, such as a potentiometer that is electrically connected to the sensor 21 to measure the voltage.

The foregoing operation of the clinical analyzer may be repeated for the different compositions in containers 30a to 30d using the same sensor 21 or different sensors like electrode 21.

### EXPERIMENTS

### METAL SPIKING

A process control solution (PCS) containing a composition similar to composition 12 but no chelating agent was filled into containers similar to a standard production lot. Thereafter, a second set of containers were filled with the PCS solution and levofloxacin was added thereto. The starting metal content of each container was verified using ICP-MS (inductively coupled plasma mass spectrometry).

The testing included adding the following ions and levels to the containers: 50 ppb, 25 ppb and 10 ppb of iron and copper ions shown in Table 1 below. The spiking experiment included adding traces of metal at the known concentrations to each container to reach the 50 ppb, 25 ppb and 10 ppb concentrations.

**TABLE 1**

| | Tested after 7 days @ 37°C | | | | | |
|---|---|---|---|---|---|---|
| | PCS lot (no levofloxacin) | | | PCS lot (levofloxacin) | | |
| Spiked Ion | 50ppb | 25ppb | 10ppb | 50ppb | 25ppb | 10ppb |
| Fe(II)1 | -23.6 | -15.3 | n/a | -5.3 | -1.0 | n/a |
| Fe(II)2 | -24.6 | -15.2 | n/a | -6.0 | -2.5 | n/a |
| Cu(II)1 | n/a | -30.5 | -16.2 | n/a | -7.2 | -4.1 |
| Cu(II)2 | n/a | -34.0 | -18.8 | n/a | -6.4 | -3.9 |

Iron Fe(II) was chosen because iron is most common metal ion contamination found in compositions due to equipment wear and tear. Copper Cu(II) was chosen due to its rapid consumption rate of oxygen at concentrations as low as 10 ppm. Fe(II) and Cu(II) were added into the containers and the containers were put in an incubator at 37°C for seven days. The oxygen decay was calculated by comparing the oxygen levels before and after the exposure to the metal ions at a temperature of 37°C. In the experiment, a decrease in partial pressure of oxygen (ρO₂) of below -5.53 mmHg (millimeters of mercury) was considered a failing result, meaning that values below -5.53 mmHg showed unacceptable oxygen depletion over seven days. As shown in Table 1 levofloxacin provided significant protection against oxidation up to 25 to 50 ppb Fe(II) and 10 ppb Cu(III).

### ANALYTE STABLITY

Container shelf-life testing was conducted to compare the shelf-life, over 10.2 months, of containers containing analyte and levofloxacin and control containers containing analyte but not containing levofloxacin. The control containers were confirmed to contain limited or no metal contamination. Storage temperatures were 15°C and 25°C. The focus of the experiment was to demonstrate that levofloxacin has no negative effect on the shelf-life of the reagents and analytes due to interactions with the analytes listed.

Table 2 below is summary of shelf-life data obtained for containers containing the compositions stored at both 15°C and 25°C. The containers with levofloxacin were shown not to have a negative effect on the analytes listed when tested at a 10.2-month period. Accordingly, the time to exceed the total allowable error (TEa) for each analyte, with a 95% confidence level, is greater than 10.2 months. The allowable TEa specifies the maximum amount of error - both imprecision and bias combined - that is allowed for an assay that must be met for patient results to be fit for use.

**TABLE 2**

| Analyte | Condition | Last time point tested (Months) |
|---|---|---|
| pH | control / no levofloxacin | 10.2 |
| | levofloxacin | 10.2 |
| pCO₂ | control / no levofloxacin | 10.2 |
| | levofloxacin | 10.2 |
| pO₂ | control / no levofloxacin | 10.2 |
| | levofloxacin | 10.2 |
| Na⁺ | control / no levofloxacin | 10.2 |
| | levofloxacin | 10.2 |
| K⁺ | control / no levofloxacin | 10.2 |
| | levofloxacin | 10.2 |
| Cl⁻ | control / no levofloxacin | 10.2 |
| | levofloxacin | 10.2 |
| Ca⁺⁺ | control / no levofloxacin | 10.2 |
| | levofloxacin | 10.2 |
| Glu | control / no levofloxacin | 10.2 |
| | levofloxacin | 10.2 |
| Lac | control / no levofloxacin | 10.2 |
| | levofloxacin | 10.2 |
| tHb | control / no levofloxacin | 10.2 |
| | levofloxacin | 10.2 |

### SENSOR STABILITY AND PERFORMANCE

To test for sensor stability and performance, a cartridge use life test was completed after one month of cartridge shelf-life and then repeated after eight months of cartridge shelf-life. Tests were performed to demonstrate that the addition of levofloxacin to the composition would not affect assay performance on a GEM^{®} Premier^{™} 5000 system.

Additionally, reagent performance on the GEM^{®} Premier^{™} 5000 system was evaluated based on drift of parameters for assays performed on the GEM^{®} Premier^{™} 5000 system. Drift for each assay parameter constitutes a deviation of measured results for each assay parameter from its factory assigned target. For example, ρO2 was measured several times during cartridge use at a defined frequency on the GEM^{®} Premier^{™} 5000, and the drift was calculated for each measurement as the difference between measured p42 for a composition and the ρO2 assigned to the composition at manufacture. This drift data was compared to demonstrate that there was no difference in pO2 performance between compositions with and without levofloxacin.

Representative sensor drift data, represented as mean and standard deviation (SD), are shown below to demonstrate that no negative effect occurred on any of the GEM^{®} Premier^{™} 5000 sensors at both the one-month and eight-month shelf-life time periods. More specifically, Table 3 shows a comparison for the one-month time period and Table 4 shows a comparison for the eight-month time period, where the composition manufactured without Levofloxacin is labeled "Control" and the composition containing levofloxacin is labeled "Levofloxacin". In this experiment, the control containers were confirmed to contain limited or no metal contamination (e.g., in parts-per-billion, cobalt 0.10, chromium 2.24, copper 0.64, iron 3.23, and manganese 0.63). The limits columns represents the maximum change that can occur during composition shelf-life according to the GEM^{®} Premier^{™} 5000 design specifications.

The data in Tables 3 and 4 shows that compositions perform similarly after one month and eight months of shelf-life with and without the addition of levofloxacin. Consequently, the data in Tables 3 and 4 shows that the addition of levofloxacin does not negatively affect sensor performance and the performance and stability of the compositions over at least eight months of shelf-life.

**TABLE 3**

| Drift at 1 Month Shelf life | | | | | |
|---|---|---|---|---|---|
| Analyte | Group | Mean | SD | Limits | Units |
| Hydroniu m | Control | 0.00 | 0.00 | 0.03 | pH |
| | Levofloxacin | 0.00 | 0.00 | | |
| pCO2 | Control | 0.20 | 0.10 | 3 | mmHg |
| | Levofloxacin | 0.11 | 0.11 | | |
| pO2 | Control | 1.48 | 2.58 | 12 | mmHg |
| | Levofloxacin | -1.70 | 1.78 | | |
| Na | Control | -0.39 | 0.26 | 5 | mmol/L |
| | Levofloxacin | -0.20 | 0.33 | | |
| K | Control | -0.03 | 0.01 | 0.5 | mmol/L |
| | Levofloxacin | 0.01 | 0.01 | | |
| Cl | Control | -0.15 | 0.37 | 6 | mmol/L |
| | Levofloxacin | -0.41 | 0.47 | | |
| Ca | Control | 0.01 | 0.00 | 0.1 | mmol/L |
| | Levofloxacin | 0.00 | 0.01 | | |
| Glu | Control | -4.24 | 3.36 | 35 | mg/dL |
| | Levofloxacin | -4.45 | 2.16 | | |
| Lac | Control | -0.22 | 0.13 | 1 | mmol/L |
| | Levofloxacin | -0.17 | 0.15 | | |
| tHb | Control | 0.01 | 0.02 | 0.5 | g/dL |
| | Levofloxacin | 0.00 | 0.03 | | |

**TABLE 4**

| Drift at 8 Months Shelf life | | | | | |
|---|---|---|---|---|---|
| Analyte | Group | Mean | SD | Limits | Units |
| Hydroniu m | Control | 0.00 | 0.00 | 0.03 | pH |
| | Levofloxacin | 0.00 | 0.00 | | |
| pCO2 | Control | 0.23 | 0.16 | 3 | mmHg |
| | Levofloxacin | 0.11 | 0.41 | | |
| pO2 | Control | -0.34 | 1.65 | 12 | mmHg |
| | Levofloxacin | -3.76 | 1.40 | | |
| Na | Control | -0.35 | 0.44 | 5 | mmol/L |
| | Levofloxacin | -0.59 | 0.30 | | |
| K | Control | 0.07 | 0.03 | 0.5 | mmol/L |
| | Levofloxacin | 0.13 | 0.04 | | |
| Cl | Control | 0.56 | 1.05 | 6 | mmol/L |
| | Levofloxacin | 0.52 | 1.11 | | |
| Ca | Control | 0.00 | 0.01 | 0.1 | mmol/L |
| | Levofloxacin | 0.00 | 0.01 | | |
| Glu | Control | -9.92 | 2.58 | 35 | mg/dL |
| | Levofloxacin | -11.04 | 2.92 | | |
| Lac | Control | -0.40 | 0.14 | 1 | mmol/L |
| | Levofloxacin | -0.37 | 0.19 | | |
| tHb | Control | 0.02 | 0.03 | 0.5 | g/dL |
| | Levofloxacin | 0.02 | 0.03 | | |

### WHOLE BLOOD

A whole blood method comparison study using native and adjusted (spiked) whole blood samples was run on the GEM^{®} Premier^{™} 5000 system and verified sensor performance and to ensure that levofloxacin does not affect the reported results.

In some implementations, the compositions described herein, such as composition 12 of Fig. 1, may be configured as a kit. For example, liquid chelating agent 16 may be packaged separately along with the aqueous liquid 13 having dissolved analytes 15 and unintended metal contaminants therein.

In some implementations, the compositions described herein, such as composition 12 of FIG. 1, may be used in a method for stabilizing oxygen in an aqueous liquid calibration reagent adapted to calibrate an oxygen sensor. An example implementation of the method includes adding one or more chelating agents 16 and an aqueous solution to a container, which includes oxygen and metal contaminants. The chelating agent 16 (e.g., a quinolone such as levofloxacin) stabilizes oxygen in the presence of the metal contaminants.

Elements of different implementations described may be combined to form other implementations not specifically set forth previously. Elements may be left out of the systems described previously without adversely affecting their operation or the operation of the system in general. Furthermore, various separate elements may be combined into one or more individual elements to perform the functions described in this specification.

Other implementations not specifically described in this specification are also within the scope of the following claims.

Further aspects, features and embodiments of the present invention are described in the following items:
1. A composition comprising:
   one or more analytes for use with an electrochemical sensor configured to detect a component of a biological sample; and
   a quinolones at a concentration of 60 milligrams/liter (mg/L) to 120 mg/L in the composition, to stabilize oxygen in a presence of trace metal.
2. The composition of item 1, wherein the quinolones comprises a fluoroquinolone.
3. The composition of item 1 or 2, wherein the quinolones comprises levofloxacin.
4. The composition of any one of the items 1 to 3, wherein the quinolones comprises at least one of: ciprofloxacin, gemifloxacin, moxifloxacin, norfloxacin, ofloxacin, pefloxacin, enoxacin, amifloxacin, tosufloxacin, difloxacin, temafloxacin, miloxacin, cinoxacin, lomefloxacin, fleroxacin, fluoroquinolones, nalidixic acid, pipemidic acid, piromidic acid, rosoxacin, oxolinic acid, flumequine, enrofloxacin, sparfloxacin, gatifloxacin, balofloxacin, clinafloxacin, or sitafloxacin.
5. The composition of any one of the items 1 to 4, further comprising:
   an aqueous liquid.
6. The composition of any one of the items 1 to 5, where the trace metal comprises one or more metals.
7. The composition of item 6, wherein the one or more metals are at a concentration in the composition of 10 parts-per-billion (ppb) or more.
8. The composition of item 5, or any one of the items 6 or 7 when dependent on item 5, wherein the aqueous liquid contains dissolved oxygen; and
   wherein the one or more analytes further comprise: pH (hydronium), carbon dioxide (CO₂), oxygen (O₂), sodium (Na), potassium (K), chlorine (Cl), calcium (Ca), magnesium (Mg), glucose, lactate, creatinine, creatinine, urea, organic dyes, amaranth, or sulforhodamine B.
9. The composition of item 8, wherein the dissolved oxygen is at a known concentration in the composition.
10. A container holding the composition of any one of the items 1 to 9.
11. The container of item 10, wherein the container comprises a bag, pouch, or glass.
12. The container of item 10 or 11, wherein the container comprises a cartridge.
13. A system comprising:
   a cartridge comprising multiple containers, each of the multiple containers holding a composition, each composition comprising:
      a liquid comprising one or more analytes; and
      a quinolones at a concentration of 60 milligrams/liter (mg/L) to 120 mg/L in the composition;
      wherein liquids in different ones of the containers have different concentrations of the one or more analytes; and
   an electrochemical sensor configured to detect at least one of the analytes, the electrochemical sensor being internal to, or external to, the cartridge.
14. The system of item 13, wherein the quinolones comprises levofloxacin.
15. The system of item 13 or 14, wherein the quinolones comprises at least one of: ciprofloxacin, gemifloxacin, moxifloxacin, norfloxacin, ofloxacin, pefloxacin, enoxacin, amifloxacin, tosufloxacin, difloxacin, temafloxacin, miloxacin, cinoxacin, lomefloxacin, fleroxacin, fluoroquinolones, nalidixic acid, pipemidic acid, piromidic acid, rosoxacin, oxolinic acid, flumequine, enrofloxacin, sparfloxacin, gatifloxacin, balofloxacin, clinafloxacin, or sitafloxacin.
16. The system of item any one of the items 13 to 15, wherein the composition comprises one or more metals, the one or more metals being at a concentration in a respective composition of 10 parts-per-billion (ppb) or more.
17. The system of item any one of the items 13 to 16, wherein the composition further comprises an aqueous liquid; and
   wherein the aqueous liquid contains dissolved oxygen, the dissolved oxygen comprising one of the analytes.
18. The system of any one of the item 13 to 17, wherein the one or more analytes further comprise: pH (Hydronium), carbon dioxide (CO₂), oxygen (O₂), sodium (Na), potassium (K), chlorine (Cl), calcium (Ca), magnesium (Mg), glucose, lactate, organic dyes, sulforhodamine 8, or amaranth,
19. A kit comprising:
   an aqueous composition comprising one or more analytes for use with an electrochemical sensor configured to detect a component of a biological sample; and
   a quinolones.
20. A method for oxygen measurement in the composition according to any one of the items 1 to 9, the method comprising;
   contacting a surface of an inert metal electrode to the composition; and reducing oxygen at the surface; and
   producing an electrical signal that is detected by a clinical analyzer;
   wherein the quinolones stabilize oxygen in the composition prior to the oxygen measurement.

## Claims

1. A composition comprising:
one or more analytes for use with an electrochemical sensor configured to detect a component of a biological sample; and
a quinolones at a concentration of 60 milligrams/liter (mg/L) to 120 mg/L in the composition, to stabilize oxygen in a presence of trace metal.

2. The composition of claim 1, wherein the quinolones comprises a fluoroquinolone, and/or
wherein the quinolones comprises levofloxacin.

3. The composition of claim 1 or 2, wherein the quinolones comprises at least one of: ciprofloxacin, gemifloxacin, moxifloxacin, norfloxacin, ofloxacin, pefloxacin, enoxacin, amifloxacin, tosufloxacin, difloxacin, temafloxacin, miloxacin, cinoxacin, lomefloxacin, fleroxacin, fluoroquinolones, nalidixic acid, pipemidic acid, piromidic acid, rosoxacin, oxolinic acid, flumequine, enrofloxacin, sparfloxacin, gatifloxacin, balofloxacin, clinafloxacin, or sitafloxacin.

4. The composition of any one of the claims 1 to 3, further comprising:
an aqueous liquid.

5. The composition of any one of the claims 1 to 4, where the trace metal comprises one or more metals,
wherein preferably the one or more metals are at a concentration in the composition of 10 parts-per-billion (ppb) or more.

6. The composition of claim 4 or claim 5 when dependent on claim 4, wherein the aqueous liquid contains dissolved oxygen; and
wherein the one or more analytes further comprise: pH (hydronium), carbon dioxide (CO₂), oxygen (O₂), sodium (Na), potassium (K), chlorine (Cl), calcium (Ca), magnesium (Mg), glucose, lactate, creatine, creatinine, urea, organic dyes, amaranth, or sulforhodamine B,
wherein preferably the dissolved oxygen is at a known concentration in the composition.

7. A container holding the composition of any one of the claims 1 to 6.

8. The container of claim 7, wherein the container comprises a bag, pouch, or glass, and/or
wherein the container comprises a cartridge.

9. A system comprising:
a cartridge comprising multiple containers, each of the multiple containers holding a composition, each composition comprising:
a liquid comprising one or more analytes; and
a quinolones at a concentration of 60 milligrams/liter (mg/L) to 120 mg/L in the composition;
wherein liquids in different ones of the containers have different concentrations of the one or more analytes; and
an electrochemical sensor configured to detect at least one of the analytes, the electrochemical sensor being internal to, or external to, the cartridge.

10. The system of claim 9, wherein the quinolones comprises levofloxacin.

11. The system of claim 9 or 10, wherein the quinolones comprises at least one of: ciprofloxacin, gemifloxacin, moxifloxacin, norfloxacin, ofloxacin, pefloxacin, enoxacin, amifloxacin, tosufloxacin, difloxacin, temafloxacin, miloxacin, cinoxacin, lomefloxacin, fleroxacin, fluoroquinolones, nalidixic acid, pipemidic acid, piromidic acid, rosoxacin, oxolinic acid, flumequine, enrofloxacin, sparfloxacin, gatifloxacin, balofloxacin, clinafloxacin, or sitafloxacin.

12. The system of any one of the claims 9 to 11, wherein the composition comprises one or more metals, the one or more metals being at a concentration in a respective composition of 10 parts-per-billion (ppb) or more.

13. The system of any one of the claims 9 to 12, wherein the composition further comprises an aqueous liquid; and
wherein the aqueous liquid contains dissolved oxygen, the dissolved oxygen comprising one of the analytes,
wherein preferably the one or more analytes further comprise: pH (Hydronium), carbon dioxide (CO₂), oxygen (O₂), sodium (Na), potassium (K), chlorine (Cl), calcium (Ca), magnesium (Mg), glucose, lactate, organic dyes, sulforhodamine 8, or amaranth.

14. A kit comprising:
an aqueous composition comprising one or more analytes for use with an electrochemical sensor configured to detect a component of a biological sample; and
a quinolones.

15. A method for oxygen measurement in the composition according to any one of the claims 1 to 6, the method comprising;
contacting a surface of an inert metal electrode to the composition; and
reducing oxygen at the surface; and
producing an electrical signal that is detected by a clinical analyzer;
wherein the quinolones stabilize oxygen in the composition prior to the oxygen measurement.
